# FASCICULE DE BREVET EUROPEEN

(11) **EP 3 207 376 B1**
(45) Date de publication et mention de la délivrance du brevet: **11.12.2019**
(21) Numéro de dépôt: 15805564.0
(22) Date de dépôt: 16.10.2015
(51) Int. Cl.: G01N 33/569

(54) **PRÉDICTION DE LA SUSCEPTIBILITÉ, POUR UN PATIENT À RISQUE, DE DÉVELOPPER OU REDÉVELOPPER UNE INFECTION À CLOSTRIDIUM DIFFICILE**
VORHERSAGE DER EMPFINDLICHKEIT EINES GEFÄHRDETEN PATIENTEN FÜR ENTWICKLUNG ODER REZIDIV VON CLOSTRIDIUM-DIFFICILE-INFEKTIONEN
PREDICTION OF THE SUSCEPTIBILITY OF AN AT RISK PATIENT TO DEVELOPING OR REDEVELOPING CLOSTRIDIUM DIFFICILE INFECTION

(30) Priorité: 17.10.2014 FR 1459996
(43) Date de publication de la demande: 23.08.2017
(73) Titulaire: Biomérieux, 69280 Marcy-L'Etoile (FR)
(72) Inventeur: FOUSSADIER, Agnès, 69007 Lyon (FR); MILLER, Mark, 69003 Lyon (FR)
(74) Mandataire: Bitaud, Valérie Marie-Odile
(86) Numéro de dépôt international: PCT/FR2015/052778
(87) Numéro de publication internationale: WO 2016/059350

(56) Documents cités:
- WO-A1-2013/009843
- M Warny ET AL: "Human antibody response to Clostridium difficile toxin A in relation to clinical course of infection", Infection and Immunity, 1 février 1994 (1994-02-01), pages 384-389, XP055191277, UNITED STATES Extrait de l'Internet: URL:http://iai.asm.org/content/62/2/384.ab stract
- P. F. JOHNSTON ET AL: "Protection from Clostridium difficile Infection in CD4 T Cell- and Polymeric Immunoglobulin Receptor-Deficient Mice", INFECTION AND IMMUNITY, vol. 82, no. 2, 1 février 2014 (2014-02-01), pages 522-531, XP055191304, ISSN: 0019-9567, DOI: 10.1128/IAI.01273-13
- X. SUN ET AL: "Mouse Relapse Model of Clostridium difficile Infection", INFECTION AND IMMUNITY, vol. 79, no. 7, 1 juillet 2011 (2011-07-01), pages 2856-2864, XP055191408, ISSN: 0019-9567, DOI: 10.1128/IAI.01336-10
- S Johnson ET AL: "Selective neutralization of a bacterial enterotoxin by serum immunoglobulin A in response to mucosal disease", Infection and Immunity, 1 août 1995 (1995-08-01), pages 3166-3173, XP055192639, UNITED STATES Extrait de l'Internet: URL:http://iai.asm.org/content/63/8/3166.a bstract
- CARROLL KAREN C. ET AL: "Biology of Clostridium difficile : Implications for Epidemiology and Diagnosis", ANNUAL REVIEW OF MICROBIOLOGY, vol. 65, no. 1, 13 octobre 2011 (2011-10-13), pages 501-521, XP055192583, ISSN: 0066-4227, DOI: 10.1146/annurev-micro-090110-102824
- S S JOHAL: "Colonic IgA producing cells and macrophages are reduced in recurrent and non-recurrent Clostridiumdifficile associated diarrhoea", JOURNAL OF CLINICAL PATHOLOGY, vol. 57, no. 9, 1 septembre 2004 (2004-09-01), pages 973-979, XP055191283, ISSN: 0021-9746, DOI: 10.1136/jcp.2003.015875
- J C Siffert ET AL: "Effects of Clostridium difficile toxin B on human monocytes and macrophages: possible relationship with cytoskeletal rearrangement", Infection and Immunity, 1 March 1993 (1993-03-01), pages 1082-1090, XP055477922, UNITED STATES Retrieved from the Internet: URL:http://iai.asm.org/content/61/3/1082.f ull.pdf

## Description

La présente invention concerne le domaine des infections par la bactérie *Clostridium difficile.* En particulier, l'invention concerne la prédiction de la susceptibilité de développer ou redévelopper des infections dues à la bactérie *Clostridium difficile.*

La bactérie *Clostridium difficile* est une bactérie toxinogène sporulée, anaérobie, de type bacille Gram+. C'est un entéropathogène à l'origine de colites pseudomembraneuses (CPM) ou de diarrhée post-antibiotiques, majoritairement impliqué dans les diarrhées nosocomiales de l'adulte. Elle est redoutable en raison de son potentiel de contagion très élevé. Bien qu'environ 5 % de la population soient porteurs asymptomatiques de la bactérie, ses manifestations pathologiques sont étroitement reliées à un séjour à l'hôpital.

Cette bactérie se développe dans une flore intestinale affaiblie par l'antibiothérapie et peut secréter deux toxines, la toxine A et la toxine B. Seules les souches produisant des toxines sont pathogènes. La toxine A, une entérotoxine, provoque l'altération de la perméabilité de l'épithélium intestinal. La toxine B, une cytotoxine, s'attaque directement aux cellules de l'épithélium. L'effet combiné des deux toxines est la diminution du temps de transit intestinal et de l'absorption intestinale, ce qui résulte en une diarrhée.

Il y a encore quelques années, la bactérie *Clostridium difficile* n'était pas considérée comme une nuisance clinique. Depuis 2003, l'émergence et la diffusion rapide de souches hypervirulentes telle que la souche *C*. *difficile* Ribotype 027 aux Etats-Unis, au Canada, puis en Europe, entrainent une vigilance de la santé publique envers cette bactérie (Kuijper E.J. et al, 2006). Aujourd'hui elle est la première cause des diarrhées infectieuses nosocomiales, responsables de 15% à 25% des colites pseudomembraneuses (Bartlett J.G., 2002). Elle est à l'origine de cas de plus en plus sévères et d'une mortalité accrue dans les centres hospitaliers. Chaque jour 7 à 12 patients sur 10000 sont touchés aux États-Unis (Freeman J. et al, 2010) et jusqu'à 5 patients sur 10000 en Europe (Bauer M.P. et al, 2011).

Le diagnostic d'une infection par *Clostridium difficile* dans les échantillons de selles est connu. Il consiste en plusieurs étapes. La première étape, qui n'est pas obligatoire, consiste à rechercher l'une des protéines détectables, représentatives de la présence de cette bactérie, à savoir la glutamate déshydrogénase (GDH). D'autres protéines détectables sont les toxines sécrétées lorsque les bactéries sont toxinogènes. La détection ou quantification de la GDH peut être mise en œuvre par immunoessai, par un test ELISA par exemple. C'est une technique qui permet d'avoir une sensibilité diagnostique plus grande que la détection ou quantification par immunoessai des toxines. En cas de positivité du test sur la GDH, une recherche des toxines est alors recommandée car cette technique présente quant à elle une plus grande spécificité.

Cette recherche de toxines dans un échantillon de selles constitue la deuxième étape, ou la première si aucune recherche de GDH n'est effectuée. Elle peut être mise en œuvre par un test ELISA ou par un test de cytotoxicité cellulaire, ce dernier constituant le « gold standard ». Il est également possible d'utiliser la PCR, qui est plus sensible. Le principal inconvénient de cette technique est qu'elle ne met en évidence que la présence des gènes des toxines A et/ou B, sans indiquer si la toxine est vraiment produite, ce qui peut être au détriment de la spécificité. En effet, la production de toxines peut être inactivée et dans ce cas la souche n'est pas pathogène.

Enfin, si la recherche de la toxine dans l'échantillon de selles est négative, une culture bactérienne est effectuée dans un milieu approprié CCFA (Cyclosérine Cefoxitine Fructose Agar), ce qui constitue la troisième étape. Si la culture est positive, on recherche des toxines sur les colonies, par exemple par test ELISA ou par test de cytotoxicité cellulaire.

Après confirmation que le patient a une infection à *Clostridium difficile,* celui-ci reçoit un traitement. Ce traitement implique des antibiotiques tels que le Métronidazole (MTZ) ou la Vancomycine (VA). Cependant, les échecs au traitement sont fréquents et les taux de récurrences atteignent les 20% lors des premiers épisodes et de 40% à 60% lors d'infections successives (C.P. Kelly et J.T. Lamont, 2008 et D.W. Eyre et al., 2012). Cette récurrence se produit normalement dans les 28 jours après la fin du traitement de l'infection primaire mais ce délai peut aller jusqu'à 6-8 semaines.

Warny M. et al (Warny M. et al., 1994) ont étudié en 1994 la réponse immunitaire humaine à la toxine A de *Clostridium difficile* chez 21 patients non-immunodéprimés ayant fait un épisode unique d'infection à *C*. *difficile* et chez 4 patients non-immunodéprimés (multi)récidivistes. Lors de cette étude, les auteurs ont observé que le titre en anticorps IgG sériques anti-toxine A et en anticorps IgA fécales anti-toxine A étaient significativement plus élevés chez les patients ayant fait un épisode unique d'infection à *Clostridium difficile* que chez les patients (multi)récidivistes. Une telle piste, obtenue sur un nombre très réduit de patients, n'a pas été poursuivie. Une autre équipe (Johnson S et al., 1992) a même obtenu des résultats inverses en ce sens qu'ils ont trouvé que les patients récidivistes avaient plutôt un titre en anticorps anti-toxine A sérique élevé.

La détermination de scores a été mise en place pour prédire les patients susceptibles de développer une infection à *Clostridium difficile.* Cette détermination de scores est basée sur plusieurs critères tels que l'âge du patient, le fait d'avoir été admis à l'hôpital, la prise d'antibiotiques, etc. (Garey K. W. et al, 2008). Toutefois les résultats de ces scores ne sont pas toujours concordants, de sorte que le clinicien n'a toujours pas accès à un outil simple tel qu'un test de diagnostic *in vitro.*

Aucune équipe n'a encore réussi à proposer un test *in vitro* qui permettrait de prédire, parmi les patients à risque, ceux susceptibles de développer ou redévelopper une infection à *Clostridium difficile.*

Il existe donc un besoin urgent, non résolu depuis de nombreuses années, de trouver de bons outils pour être capable de prédire, chez les patients à risque, ceux risquant de développer ou redévelopper une infection à *Clostridium difficile.*

La Demanderesse a découvert contre toute attente qu'il était possible d'utiliser les anticorps de type IgA fécales, dirigés contre la toxine B de *Clostridium difficile,* comme marqueur pour identifier de tels patients à risque.

Aussi, l'invention concerne un procédé de prédiction de la susceptibilité, pour un patient à risque, de développer ou redévelopper une infection à *Clostridium difficile,* comprenant ou consistant à déterminer par immunoessai, dans un échantillon de selles issu dudit patient, le taux d'anticorps IgA anti-toxine B de *Clostridium difficile,* et à comparer ce taux à une valeur de référence S préalablement déterminée avec deux populations de patients exposés à la bactérie, l'une n'ayant pas développé ou redéveloppé une telle infection et l'autre ayant développé ou redéveloppé une telle infection,
- un taux inférieur à ladite valeur de référence S signifiant que le patient est un patient à risque accru de développer ou redévelopper une infection à *Clostridium difficile,* et
- un taux supérieur à ladite valeur de référence S signifiant que le patient n'est pas un patient à risque accru de développer ou redévelopper une infection à *Clostridium difficile.*

Elle concerne également un procédé de détermination, par immunoessai, du taux d'au moins un anticorps IgA dirigé contre une protéine non affectée en présence d'acide, de préférence dirigé contre la toxine B de *Clostridium difficile,* dans un échantillon biologique d'un patient susceptible de contenir ledit au moins un anticorps IgA. Le procédé comprend ou consiste en la mise en présence d'un ou plusieurs partenaires de liaison audit au moins un anticorps IgA, utilisé(s) pour la mise en œuvre de l'immunoessai, avec un milieu réactionnel acide comprenant ledit échantillon biologique prétraité par un tampon de traitement d'échantillon acide, sans neutralisation avant sa mise en œuvre dans l'immunoessai.

L'invention concerne enfin une trousse pour la détermination par immunoessai du taux d'au moins un anticorps IgA dirigé contre une protéine non affectée en présence d'acide, notamment dirigé contre la toxine B de *Clostridium difficile,* dans un échantillon biologique d'un patient susceptible de contenir ledit au moins un IgA, notamment un échantillon de selles, comprenant (i) un ou des partenaires de liaison audit au moins un anticorps IgA pour la mise en œuvre de l'immunoessai, et (ii) un tampon de traitement d'échantillon acide, étant entendu que ladite trousse ne contient aucune solution de neutralisation.

La Demanderesse a donc montré, contre toute attente, qu'il était possible de prédire la susceptibilité, chez un patient à risque, de développer ou redévelopper une infection à *Clostridium difficile* en déterminant, par immunoessai, dans un échantillon de selles issu dudit patient, le taux d'anticorps IgA anti-toxine B de *Clostridium difficile.*

Par développer une infection à *Clostridium difficile,* on entend que le patient développe pour la première fois l'infection, quelle que soit la souche de *Clostridium difficile.*

Par redévelopper une infection à *Clostridium difficile,* on entend que le patient fait une récurrence, c'est-à-dire une récidive (infection due à la même la souche de *Clostridium difficile*) ou une réinfection (infection due à une souche de *Clostridium difficile* différente de la souche à l'origine de l'infection primaire).

Par patient à risque, on entend une personne vulnérable. Ce patient peut être vulnérable du fait de son état, par exemple parce que :
- il est immunodéprimé ou susceptible de devenir immunodéprimé suite à un traitement médicamenteux futur ou à la survenue d'une nouvelle maladie ;
- il vient de développer une infection, quel que soit le microorganisme, excepté *Clostridium difficile* ; il a pu recevoir dans ce cadre un traitement antibiotique ;
- il vient de développer une infection à *Clostridium difficile* ; il a pu recevoir dans ce cadre un traitement antibiotique. Dans ce cas, ce patient pourrait redévelopper l'infection à *Clostridium difficile.*

Il peut être aussi vulnérable du fait de son âge, parce que c'est un prématuré, un nourrisson ou une personne de plus de 65 ans.

Il peut aussi être vulnérable du fait de son environnement car il se trouve, ou va se trouver, dans un lieu dans lequel les bactéries *Clostridium difficile* peuvent être présentes, tel qu'à l'hôpital ou dans un centre de soins.

Bien entendu, une personne peut n'avoir qu'un de ces risques de vulnérabilité ou les cumuler.

A titre d'exemples non limitatifs de patients à risque, on peut citer les personnes venant de faire une infection à *Clostridium difficile,* lesquelles sont encore présentes à l'hôpital. Dans ce cas, le procédé de l'invention peut être effectué à la fin du traitement (après environ 7-10 jours de traitement et jusqu'à 28 jours après la fin du traitement). Selon un mode de réalisation, le patient à risque est un patient diagnostiqué comme ayant fait une infection à *Clostridium difficile,* et le risque consiste à redévelopper une nouvelle infection à *Clostridium difficile.*

On peut également citer les personnes connues ou non pour avoir fait une infection à *Clostridium difficile,* et arrivant à l'hôpital, ces personnes risquant de se retrouver sous traitement antibiotique et/ou sous immunosuppresseurs. Selon un autre mode de réalisation, le patient à risque est un patient arrivant à l'hôpital et étant susceptible de recevoir un médicament entraînant une diminution de ses défenses immunitaires.

On peut également citer les personnes arrivant à l'hôpital, étant âgées de plus de 65 ans, ou étant immunodéprimées, et risquant de se retrouver sous traitement antibiotique et/ou immunosuppresseurs, quel que soit le statut de ces personnes pour l'infection à *Clostridium difficile,* c'est-à-dire n'ayant jamais eu d'infection à *Clostridium difficile,* étant porteur de *Clostridium difficile* ou ayant déjà eu une infection à *Clostridium difficile.*

Selon le résultat du procédé de l'invention et la stratification du patient, s'il est considéré comme étant un patient à risque accru de développer ou redévelopper une infection à *Clostridium difficile,* le clinicien pourra décider d'une ou plusieurs des actions suivantes : prise d'un traitement antibiotique modéré, isolement du patient pour éviter toute contamination, report du traitement immunosuppresseur, prise d'une immunothérapie préventive, vaccination.

Le procédé de prédiction de la susceptibilité, pour un patient à risque, de développer ou redévelopper une infection à *Clostridium difficile* met en œuvre un immunoessai. L'immunoessai est un essai largement connu de l'homme du métier. En quelques mots, il consiste à déterminer le taux d'analyte, dans le cas présent les anticorps IgA anti-toxine B de *Clostridium difficile,* en mettant en œuvre au moins un partenaire de liaison à cet analyte.

Bien entendu, le préfixe « immuno » dans le terme « immunoessai », par exemple, n'est pas à considérer dans la présente demande comme indiquant strictement que le partenaire de liaison est nécessairement un partenaire d'origine immunologique, tel qu'un anticorps ou un fragment d'anticorps. En effet, comme cela est bien connu de l'homme du métier, ce terme est plus largement utilisé pour désigner aussi des tests et procédés dans lesquels le partenaire de liaison n'est pas un partenaire d'origine/de nature immunologique mais consiste, par exemple, en un récepteur de l'analyte que l'on souhaite détecter et/ou quantifier. La condition essentielle est que le partenaire de liaison concerné soit capable de se lier à l'analyte recherché, dans le cas présent de nature anticorps, de préférence de manière spécifique. Ainsi, il est connu de parler de l'essai ELISA pour des essais qui utilisent des partenaires de liaison non immunologiques *stricto sensu,* appelés plus largement en anglais « ligand binding assay », que l'on pourrait traduire en langue française par « essai utilisant la liaison à un ligand », alors que le terme « immuno » est inclus dans l'intitulé *in extenso* correspondant à l'acronyme ELISA. Dans un souci de clarté et d'uniformité, le terme « immuno » est employé dans la présente demande pour désigner toute analyse biologique utilisant au moins un partenaire de liaison adapté pour se lier à l'analyte recherché et détecter et/ou quantifier ce dernier, de préférence de manière spécifique, même quand ledit partenaire de liaison n'est pas de nature ou d'origine immunologique au sens strict.

Par partenaire de liaison aux anticorps IgA dirigés contre la toxine B de *Clostridium difficile,* également appelé anticorps IgA anti-toxine B de *Clostridium difficile,* on entend toute molécule capable de se lier auxdites IgA. A titre d'exemple de partenaire de liaison aux IgA anti-toxine B, on peut citer la toxine B native ou recombinante, des fragments de la toxine B, les anticorps anti-IgA ou toute autre molécule qui est connue pour avoir une interaction avec les IgA anti-toxine B.

La toxine B d'origine naturelle, ou encore appelée native, peut être obtenue après culture de la bactérie *Clostridium difficile* et purification de la protéine à partir du lysat bactérien. La toxine B recombinante peut être obtenue par génie génétique, par des techniques bien connues de l'homme du métier. Une telle obtention est décrite par exemple par Anderson BM *et al*., 1993. La toxine B native peut être obtenue auprès de Sociétés telles que the Native Antigen Company (Upper Heyford, Royaume-Uni).

Les partenaires de liaison de type anticorps sont par exemple soit des anticorps polyclonaux, soit des anticorps monoclonaux, dont l'obtention est largement connue de l'homme du métier.

A titre d'exemple de fragments d'anticorps, on peut citer les fragments Fab, Fab', F(ab')2 ainsi que les scFv (Single chain variable fragment), dsFv (Double-stranded variable fragment). Ces fragments fonctionnels peuvent notamment être obtenus par génie génétique.

L'immunoessai consistant à déterminer le taux d'IgA anti-toxine B est un essai semi-quantitatif ou quantitatif largement connu de l'homme du métier mettant en œuvre de préférence deux partenaires de liaison aux IgA. L'un des deux partenaires peut être couplé à un marqueur pour former un conjugué ou un traceur. L'autre partenaire de liaison peut être capturé sur un support solide. On parle alors de partenaire de capture pour ce dernier et partenaire de détection pour le premier. De préférence, le partenaire de capture est la toxine B de *Clostridium difficile* et le partenaire de détection est un anticorps anti-IgA humain.

Le signal mesuré émis lors de l'immunoessai est alors proportionnel à la quantité d'IgA anti-toxine B de l'échantillon biologique.

Par marqueur, on entend, notamment, toute molécule contenant un groupement réactif avec un groupement du partenaire de liaison, directement sans modification chimique, ou après modification chimique pour inclure un tel groupement, laquelle molécule est capable de générer directement ou indirectement un signal détectable. Une liste non limitative de ces marqueurs de détection directe consiste en :
- les enzymes qui produisent un signal détectable par exemple par colorimétrie, fluorescence, luminescence, comme la peroxydase de raifort, la phosphatase alcaline, la β-galactosidase, la glucose-6-phosphate déshydrogénase,
- les chromophores comme les composés fluorescents, luminescents, colorants,
- les molécules radioactives comme le ³²P, le ³⁵S ou le ¹²⁵I,
- les molécules fluorescentes telles que les Alexa ou les phycocyanines, et
- les sels électrochimiluminescents tels que des dérivés organo-métalliques à base d'acridinium ou de ruthénium.

Des systèmes indirects de détection peuvent aussi être utilisés, comme par exemple des ligands capables de réagir avec un anti-ligand. Le ligand correspond alors au marqueur pour constituer, avec le partenaire de liaison, le conjugué.

Les couples ligand/anti-ligand sont bien connus de l'homme du métier, ce qui est le cas par exemple des couples suivants : biotine/streptavidine, haptène/anticorps, antigène/anticorps, peptide/anticorps, sucre/lectine, polynucléotide/complémentaire du polynucléotide.

L'anti-ligand peut alors être détectable directement par les marqueurs de détection directe décrits précédemment ou être lui-même détectable par un autre couple ligand/anti-ligand, et ainsi de suite.

Ces systèmes indirects de détection peuvent conduire, dans certaines conditions, à une amplification du signal. Cette technique d'amplification du signal est bien connue de l'homme du métier, et l'on pourra se reporter aux demandes de brevet antérieures FR 2781802 ou WO 95/08000 de la Demanderesse.

Selon le type de marquage utilisé, l'homme du métier ajoutera des réactifs permettant la visualisation du marquage ou l'émission d'un signal détectable par tout type d'appareil de mesure approprié, comme par exemple un spectrophotomètre, un spectrofluorimètre, un densitomètre ou encore une caméra haute définition.

L'immunoessai peut également comprendre d'autres étapes connues de l'homme du métier, telles que des étapes de lavage et des étapes d'incubation.

L'immunoessai peut être un essai en une étape ou en deux étapes, comme cela est largement connu de l'homme du métier. En quelques mots, un immunoessai en une étape comprend la mise en présence de l'échantillon à tester simultanément avec les deux partenaires de liaison, alors qu'un immunoessai en deux étapes comprend la mise en présence de l'échantillon à tester d'une part avec le premier partenaire de liaison, puis le complexe analyte-premier partenaire de liaison ainsi formé est mis en présence du deuxième partenaire de liaison.

La valeur de référence S utilisée dans le procédé selon l'invention est une valeur obtenue au préalable avec deux populations de patients exposés à la bactérie *Clostridium difficile,* par exemple dans un milieu hospitalier, l'une n'ayant pas développé ou redéveloppé une telle infection et l'autre ayant développé ou redéveloppé une telle infection. Une telle détermination est largement connue de l'homme du métier. Elle consiste notamment à mettre en œuvre un immunoessai identique à celui mis en œuvre dans le procédé de l'invention, dans des échantillons de selles de ces deux populations, et à déterminer la valeur du test (signal) permettant de faire une discrimination entre ces deux populations.

Lorsque la valeur de référence S est une quantité, un titre ou une concentration d'anticorps, l'immunoessai mis en œuvre est quantitatif et le résultat du procédé donne la quantité, le titre ou la concentration en anticorps IgA. Il convient alors de corréler le signal obtenu à la quantité, au titre ou à la concentration dans l'échantillon biologique en utilisant un modèle mathématique préétabli à partir d'une gamme étalon. Cette gamme étalon sera obtenue préalablement de façon connue. En quelques mots, l'obtention d'une gamme étalon consiste à mesurer le signal généré par des quantités ou concentrations croissantes et connues de l'anticorps IgA anti-toxine B, à tracer la courbe donnant le signal en fonction de la quantité, du titre ou de la concentration et à trouver un modèle mathématique qui représente de la manière la plus fidèle possible cette relation. Le modèle mathématique sera utilisé pour déterminer par extrapolation les quantités, titres ou concentrations d'anticorps IgA anti-toxine B inconnues, contenues dans l'échantillon biologique à tester.

Les échantillons biologiques susceptibles de contenir les IgA anti-toxine B de *Clostridium difficile* utiles au sens de l'invention sont les échantillons de selles issus de patients. Ce peut être les selles elles-mêmes ou un lavement rectal. Ces échantillons peuvent être utilisés tels quels dans le procédé de l'invention ou avoir subi un prétraitement, ce dernier mode de réalisation étant préféré.

Les échantillons de selles sont une matrice difficile à traiter. Les IgA peuvent être impliquées dans des complexes constitués de mucus, de protéines, de toxines, de particules, etc. Selon un mode de réalisation, les échantillons de selles mises en œuvre dans l'immunoessai ont été traités préalablement par un tampon de traitement d'échantillon acide. Par tampon de traitement d'échantillon acide, on entend un tampon ayant un pH compris entre 1,5 et 3, de préférence entre 2 et 3, un pH de 2,5 étant préféré.

Comme exemple de tampon de traitement d'échantillon acide approprié aux fins de l'invention, on peut citer le tampon acide chlorhydrique-glycine (pH 2,2-3), le tampon phosphate-citrate (pH 2,2-3), le tampon acide chlorhydrique-chlorure de potassium (pH 2-2,2) et le tampon acide citrique-citrate de sodium (pH 3).

La durée du prétraitement des échantillons de selles doit être raisonnable, une durée d'au plus 30 min étant appropriée. Selon un mode de réalisation, la durée de prétraitement des échantillons de selles ou de lavement rectal est d'au plus 30 min, de préférence d'au plus 20 min, 15 min, 10 min, 5 min, 3 min, 2 min, 1 min, 45 s, 30 s, 15 s ou 10 s.

L'échantillon biologique ainsi traité est alors soumis à une séparation, notamment par filtration ou sédimentation, par exemple une centrifugation, le filtrat ou le surnageant, contenant les IgA d'intérêt, étant alors récupéré pour sa mise en œuvre dans l'immunoessai du procédé de l'invention.

De façon habituelle, lorsqu'un échantillon biologique subit un prétraitement acide, le milieu réactionnel ainsi obtenu est ensuite neutralisé pour faire remonter le pH avant sa mise en œuvre dans un immunoessai, et ce pour éviter l'altération des partenaires de liaison utilisés pour l'immunoessai, notamment des partenaires de capture. Contre toute attente, une telle étape de neutralisation n'est pas nécessaire dans le procédé de l'invention de sorte que, selon un mode de réalisation, l'étape de prétraitement de l'échantillon n'inclut pas d'étape de neutralisation du milieu réactionnel avant sa mise en œuvre dans l'immunoessai.

Par étape de neutralisation du milieu réactionnel, on entend l'ajout d'une solution de neutralisation, basique pour une solution à neutraliser acide, permettant de faire remonter le pH aux alentours de 7 afin de ne pas altérer les partenaires de liaison mis en œuvre dans l'immunoessai. Dans le cadre de l'invention, cette étape n'est donc pas nécessaire.

Il est à noter que tous les autres tampons utilisés classiquement utilisés dans un immunoessai, tels que les tampons de lavage ou les tampons réactionnels, ne sont pas des solutions de neutralisation puisque leur but n'est pas de faire remonter le pH du milieu réactionnel acide aux alentours de 7.

D'autres marqueurs peuvent être utilisés pour aider à la prédiction de la susceptibilité, pour un patient à risque, de développer ou redévelopper une infection à *Clostridium difficile.* De tels marqueurs sont par exemple les anticorps IgA anti-toxine A de *Clostridium difficile.*

La détermination du taux d'anticorps IgA anti-toxine A de *Clostridium difficile* peut être mise en œuvre de façon similaire à celle décrite pour la détermination du taux d'anticorps anti-toxine B : dans un échantillon de selles, le cas échéant prétraité par un tampon de traitement d'échantillon acide, en mettant en œuvre un immunoessai.

Comme nous venons de le voir, un immunoessai consistant à déterminer un taux d'IgA dans un échantillon biologique peut être réalisé dans un milieu réactionnel acide, contre toute attente. Aussi, un autre objet de l'invention consiste en un procédé de détermination, par immunoessai, du taux d'au moins un anticorps IgA dans un échantillon biologique d'un patient susceptible de contenir ledit au moins un anticorps IgA, comprenant la mise en présence d'un ou plusieurs partenaires de liaison audit au moins un anticorps IgA, utilisé(s) pour la mise en œuvre de l'immunoessai, avec un milieu réactionnel acide comprenant ledit échantillon biologique prétraité par un tampon de traitement d'échantillon acide, sans neutralisation avant sa mise en œuvre dans l'immunoessai.

En d'autres termes, le procédé de détermination, par immunoessai, du taux d'au moins un anticorps IgA dans un échantillon biologique d'un patient susceptible de contenir ledit au moins un anticorps IgA, comprend ou consiste en :
- la disposition d'un échantillon biologique d'un patient prétraité par un tampon de traitement d'échantillon acide,
- la disposition d'une trousse d'immunoessai comprenant un ou plusieurs partenaires de liaison audit au moins un anticorps IgA,
- la mise en contact dudit échantillon prétraité avec le ou les partenaires de liaison de ladite trousse, étant entendu que ledit échantillon prétraité n'est pas soumis à une neutralisation avant son contact avec le ou les partenaires de liaison, et
- la détermination du taux d'anticorps IgA.

Les anticorps IgA appropriés au sens de l'invention sont les anticorps dirigés contre une protéine non affectée en présence d'acide. En effet, afin que le procédé soit spécifique, il convient d'utiliser, comme partenaire de liaison à l'anticorps IgA au moins la protéine que reconnait l'anticorps, ou un de ses fragments. Et cette protéine, mise en présence de l'échantillon prétraité, donc à pH acide, doit résister à cette condition acide.

Par protéine non affectée en présence d'acide, on entend toute protéine dont la structure tridimensionnelle est peu ou pas altérée à pH acide. Cette stabilité, qui correspond à une résistance à la dénaturation acide, peut être démontrée par toute méthode connue, comme par exemple par microcalorimétrie de type DSC (Differential Scanning Calorimetry ou Calorimétrie à Balayage Différentielle). En quelques mots, cette méthode consiste à forcer la dénaturation thermique d'une protéine au sein d'un calorimètre dans un milieu ayant le pH voulu, et déduire les deux paramètres que sont le Tm et le ΔH à partir de mesures effectuées. La Tm est un paramètre semi-empirique, qui correspond à la température à laquelle 50% de la protéine est à l'état natif et 50% à l'état dénaturé. Le ΔH (variation d'enthalpie, ou chaleur de dénaturation) est un paramètre quantitatif correspondant à l'énergie nécessaire à fournir à la protéine pour obtenir sa dénaturation totale. Ces deux paramètres sont en lien direct avec la stabilité intrinsèque de la protéine testée, plus leurs valeurs respectives croissent, plus la stabilité de la protéine est élevée par rapport à une valeur référence.

Pour déterminer si la structure tridimensionnelle d'une protéine est affectée ou pas en présence d'acide, il faut déterminer les Tm et/ou ΔH de cette protéine à pH acide et à son pH de stabilité immunologique. Dans cette expérience comparative, le pH est l'unique facteur variable. La concentration de la protéine à étudier, la vitesse de chauffage et la nature chimique du tampon doivent tous être constants. Il convient ainsi de choisir des agents tampon à plusieurs pKa permettant une large gamme d'effet tamponnant, tels le phosphate (1.7 - 2.9 ; 5.8 - 8.0), le citrate (2.2 - 6.5) ou le succinate (3.2 - 5.2 ; 5.5 - 6.5), ou l'emploi d'un tri-tampon pouvant couvrir une gamme pH3 - pH10. Le pH acide à choisir pour cette expérience comparative est le pH du tampon de traitement d'échantillon acide selon l'invention. On considère que la structure de la protéine n'est pas affectée en présence d'acide lorsque le ΔH déterminé à pH acide ne diffère pas d'au plus environ 25%, de préférence d'au plus environ 10%, par rapport au ΔH déterminé au pH de stabilité immunologique de la protéine. Si c'est la Tm qui a été calculée, la Tm déterminée à pH acide ne doit pas différer d'au plus environ 25%, de préférence d'au plus environ 10%, par rapport à la Tm déterminée au pH de stabilité immunologique de la protéine étudiée.

Les protéines non affectées en présence d'acide sont largement connues de l'homme du métier. A titre d'exemple, on peut citer les protéines de microorganismes, telles que celles de bactéries et virus, en particulier de structures bactériennes et virales, à l'exclusion des enveloppes, par exemple la protéine Core du virus HCV, ainsi que les toxines telles que les toxines A et B de *Clostridium difficile.*

Les caractéristiques décrites précédemment dans le cadre du procédé de prédiction de la susceptibilité, pour un patient à risque, de développer ou redévelopper une infection à *Clostridium difficile,* s'appliquent au procédé de détermination du taux d'IgA décrit ici, comme par exemple l'immunoessai lui-même, la nature du tampon de traitement d'échantillon acide, son pH, le fait d'utiliser ou non une séparation par filtration ou par sédimentation du milieu réactionnel après traitement acide.

En particulier, comme précédemment, la durée du prétraitement doit être raisonnable, notamment de moins de 30 min, contrairement aux enseignements de Tress U. et al., 2006 qui préconisent un traitement d'une nuit d'échantillons de selles de chien pour la quantification d'IgA totaux et une étape de neutralisation avant l'immunoessai.

Tous les anticorps IgA représentant un intérêt diagnostic ou pronostic et dirigés contre une protéine non affectée en présence d'acide, y compris ceux dirigés contre la toxine B de *Clostridium difficile,* de préférence secrétoires, sont appropriés aux fins de ce procédé. Par ailleurs, des échantillons biologiques autres que les selles et le lavement rectal, tels que les mucus, le crachat, le lavage broncho alvéolaire, les sécrétions vaginales, le lavement vaginal, la sueur, les larmes, la salive, le lait, le colostrum, sont également appropriés.

Les trousses pour la détermination par immunoessai du taux d'au moins un anticorps IgA dirigé contre une protéine non affectée en présence d'acide, notamment des anticorps IgA anti-toxine B de *Clostridium difficile,* dans un échantillon biologique d'un patient susceptible de contenir ledit au moins un IgA, comprenant ou contenant (i) un ou des partenaires de liaison audit au moins un anticorps IgA pour la mise en œuvre de l'immunoessai, et (ii) un tampon de traitement d'échantillon acide, de préférence à pH 2,5, étant entendu que ladite trousse ne contient aucune solution de neutralisation, constituent un autre objet de l'invention.

Les composants et caractéristiques de cette trousse sont tels que définis précédemment.

Selon un mode de réalisation particulier, les trousses comprennent ou contiennent également au moins un échantillon contrôle qui est un échantillon contenant une quantité connue d'anticorps IgA anti-toxine B de *Clostridium difficile.*

Les trousses peuvent également contenir tous les composés nécessaires pour la mise en évidence de la réaction entre le ou les partenaires de liaison et les anticorps IgA cibles, tels que des tampons de lavage ou des réactifs permettant la visualisation d'un marquage ou l'émission d'un signal détectable.

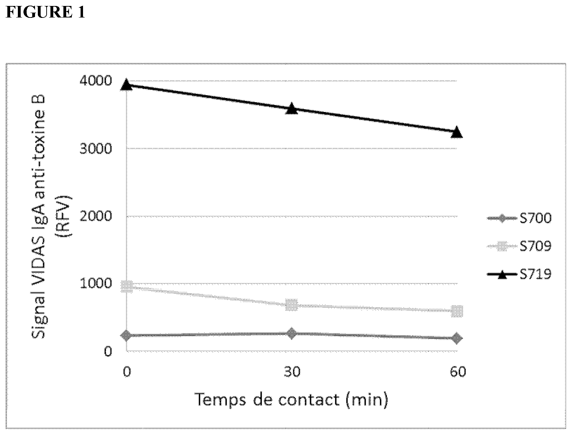

L'invention sera mieux comprise à l'aide des exemples suivants qui sont donnés à titre illustratif et non limitatif, ainsi qu'à l'aide de la figure 1 qui représente un graphe donnant le signal RFV obtenu en utilisant l'instrument VIDAS®, correspondant au taux d'anticorps IgA anti-toxine B de 3 échantillons de selles (S719- échantillon positif élevé ; S709- échantillon positif faible ; et S700- échantillon négatif) mis en contact préalablement avant l'immunoessai avec un tampon de traitement d'échantillon acide, en fonction de la durée du contact entre l'échantillon et le tampon de traitement d'échantillon acide.

### EXEMPLES

### Exemple 1 : Préparation des toxines A et B recombinantes de Clostridium difficile

Le gène *tcdA* codant pour la toxine A de *Clostridium difficile* et le gène *tcdB* codant pour la toxine B de *Clostridium difficile* sont issus de la souche de référence VPI 10463, toxinotype 0, ribotype 087 (toxine A : n° d'accession Swissprot : P16154 et toxine B : n° d'accession Swissprot : P18177). Pour chacun des gènes, l'intégralité de la séquence a été optimisée par Geneart (Invitrogen) pour une expression dans *E. coli* et 8 Histidines ont été ajoutées côté N-term pour permettre la purification par chromatographie d'affinité métal-chélate. Les gènes synthétiques obtenus ont été clonés dans le vecteur pET3d (Novagen) à l'aide des sites de restriction Nco1 et BamH1.

Les plasmides d'expression ainsi construits sont introduits dans des bactéries *E*. *coli* BL21 et dérivées (Stratagene, Agilent Technologies). Les cultures sont réalisées en milieu 2x YT (Difco), en présence d'ampicilline 100 µg/mL et glucose 10%, à 24°C sous agitation. L'induction de l'expression de la protéine se fait par addition de 1 mM d'IPTG (isopropyl beta-D-1-thiogalactopyranoside) pendant 4h, une fois que l'on est en phase de croissance exponentielle. En fin de culture, les bactéries sont collectées par centrifugation à 10000 g, à 4°C, pendant 30 min. Les culots bactériens sont congelés à -80°C.

Les culots bactériens sont repris en tampon PBS 2X (phosphate buffered saline) et lysés. Les lysats sont centrifugés à 3000 g pendant 30 min à 4°C. Le surnageant contient les protéines solubles purifiées, la toxine A ou la toxine B recombinante selon le plasmide d'expression utilisé initialement.

La purification des protéines se fait par chromatographie d'affinité métal chélate en une étape. Le surnageant obtenu après centrifugation est chargé sur une résine Ni-NTA-Agarose (Qiagen). Après un cycle de lavage, la protéine (toxine A ou toxine B) est éluée en présence d'imidazole 250 mM. La protéine est dialysée dans un tampon phosphate 50 mM, NaCl 150 mM.

### Exemple 2 : Immunoessai pour la détection des IgA fécales anti-toxine B

Les immunoessais ont été réalisés en utilisant l'automate d'immunoanalyse VIDAS® (bioMérieux). Le cône à usage unique sert à la fois de phase solide pour la réaction et de système de pipetage. La cartouche est composée de 10 puits (X0 à X9) recouverts d'une feuille d'aluminium scellée et étiquetée. Le premier puits (X0) comporte une partie prédécoupée pour faciliter l'introduction de l'échantillon. Le dernier puits (X9) est une cuvette optique dans laquelle la fluorescence du substrat est mesurée. Les différents réactifs nécessaires à l'analyse sont contenus dans les puits intermédiaires (X1 à X8). Toutes les étapes du test sont réalisées automatiquement par l'instrument. Elles sont constituées d'une succession de cycles d'aspiration/refoulement du milieu réactionnel.

### a) Sensibilisation et passivation des cônes

Les cônes ont été sensibilisés avec 300 µL d'une solution de toxine B recombinante ou native diluée à 2 µg/mL dans un tampon PBS, pH 7,2. La toxine B native inactivée (Cat. No. CDB-TDL) a été obtenue auprès de la société the Native Antigen Company (Upper Heyford, Royaume-Uni). Après environ 20h d'incubation à +18/25°C avec la solution de sensibilisation, les cônes sont vidés. Ensuite, 300 µL d'une solution de Tris 200 mM contenant de 5 g/L d'albumine bovine sont ajoutés. La passivation se poursuit à +18/25°C sur la nuit. Les cônes sont vidés, séchés, puis conservés à +4°C jusqu'à utilisation, à l'abri de l'humidité.

### b) Pré-traitement de l'échantillon

Un volume de selles est mis en contact avec 3 volumes d'un tampon de traitement d'échantillon (dilution 1/4). L'amélioration de la composition de ce tampon est illustrée dans l'exemple 3. Une homogénéisation manuelle suivie d'une agitation au vortex est réalisée. Cette étape dure environ entre 30 secondes et 2 minutes, selon la consistance des selles. Les échantillons sont centrifugés pendant 5 minutes à 12000 g afin de récupérer les protéines solubles. L'immunoessai est réalisé sur ce surnageant qui est déposé directement dans le puits X1 de la cartouche VIDAS®.

### c) Mode opératoire de l'immunoessai

Le puits X1 contient 300 µL de diluant échantillon dont la composition est identique au tampon de traitement d'échantillon. 200 µL du surnageant obtenu à l'étape b) sont transférés dans le puits X1 afin réaliser une dilution supplémentaire. Dès que le cône VIDAS® est en contact avec l'échantillon, la première étape de la réaction immunologique commence. Cette étape permet la liaison spécifique des IgA fécales anti-toxine B, présents ou non dans le surnageant d'échantillon de selles, à la toxine B adsorbée sur le cône. Après 4 minutes d'incubation à 37°C, les composants non liés sont éliminés par lavages avec un tampon Tris 200 mM pH 7,8, NaCl 300 mM, Tween® 20 0,25%. Lors de la seconde étape, le cône est incubé avec une solution de conjugué contenant environ 0,5 µg/mL d'une IgG de souris anti-IgA humain (bioMérieux) couplé à la phosphatase alcaline, dans un tampon phosphate 10 mM, contenant 300 mM de NaCl et 5 g/L d'albumine sérique bovine. Le puits X5 contient 400 µL de cette solution que le cône aspire/refoule pendant 5 minutes, toujours à 37°C. La seconde étape entraine la formation d'un complexe entre les IgA fécales anti-toxine B et le conjugué anti-IgA couplé à la phosphatase alcaline. Cette étape est suivie de 3 lavages successifs afin d'éliminer les composés non fixés.

Lors de l'étape finale de révélation, le substrat 4-méthylombelliferyl phosphate est aspiré puis refoulé dans le cône ; l'enzyme du conjugué catalyse la réaction d'hydrolyse de ce substrat en 4-méthylombelliferone dont la fluorescence émise est mesurée à 450 nm. La valeur du signal de fluorescence (RFV=relative fluorescence value) est proportionnelle à la concentration de l'IgA fécale anti-toxine B présente dans l'échantillon.

Le Tableau 1 ci-dessous récapitule les signaux de fluorescence (RFV=relative fluorescence value) déterminés par l'automate VIDAS® en utilisant en tant que tampon de traitement d'échantillon le tampon R1. Ce tampon fait partie du kit VIDAS® *C. difficile* Toxine A&B. (Cat. No. 30118, bioMérieux) qui permet la détection des toxines A et B dans les selles. Son pH est de 7,2, comme décrit dans la notice du kit. Il est utilisé pour extraire les toxines des selles humaines. Les échantillons de selles utilisés ont été obtenus chez des patients ayant consulté dans le service du Dr. Robert Spencer, Heath Protection Agency Regional Laboratory, Bristol, Royaume-Uni avec une suspicion d'infection à *Clostridium difficile.*

**Tableau 1. Détection des IgA fécales dans les selles humaines.**

| **Code Echantillon** | **IgA anti-toxine B Signal VIDAS® (RFV)** | **IgA anti-toxine B Interprétation du Test** | **VIDAS® GDH** | **VIDAS® Tox A & B** |
|---|---|---|---|---|
| **S700** | 36 | Neg | Neg | ND |
| **S706** | 9 | Neg | Neg | ND |
| **S709** | 114 | Pos | Neg | ND |
| **S713** | 91 | Neg | Pos | Neg |
| **S719** | 831 | Pos | Neg | ND |
| **S720** | 56 | Neg | Pos | Neg |

| | | | | |
|---|---|---|---|---|
| Neg = négatif ; Pos = positif ; ND = non déterminé | | | | |

L'interprétation du résultat de l'immunoessai est réalisée en comparant le signal RFV mesuré pour chaque échantillon à la limite de détection du test VIDAS® qui est définie ainsi : bruit de fond moyen (signal RFV mesuré avec un tampon) + 3 écart-types. Les signaux VIDAS® supérieurs à cette limite de détection sont considérés comme positifs : il y a présence d'anticorps IgA anti-toxine B. Les signaux VIDAS® inférieurs à cette limite de détection sont considérés comme négatifs : il y a absence d'anticorps IgA anti-toxine B.

Il est important de noter que l'échantillon S709 avec un signal de 114 RFV est classé comme positif alors que l'échantillon S713 avec un signal de 91 RFV est classé comme négatif. Deux signaux qui sont proches en valeur absolue aboutissent à une interprétation biologique différente : cette situation n'est pas satisfaisante. Il convient donc de l'améliorer.

### Exemple 3 : Optimisation de l'extraction des IgA fécales

Le tampon R1 adapté à l'extraction des toxines A et B, utilisé dans l'exemple 2 a montré qu'il était possible d'améliorer le dosage des IgA fécales. Un autre tampon de traitement d'échantillon a donc été testé en utilisant les échantillons de selles caractérisés dans l'exemple 2, tout en conservant le temps de traitement (30 s et 2 min). Ce tampon est un tampon acide chlorhydrique- Glycine à ph 2,5 (Glycine 1M pH 2,5). Le Tableau 2 ci-dessous récapitule les signaux de fluorescence (RFV=relative fluorescence value) déterminés par l'automate VIDAS®. Les résultats présentés dans cette expérience ont été obtenus en utilisant des cônes sur lesquels la toxine B recombinante a été immobilisée.

**Tableau 2. Détection des IgA fécales extraites avec différents tampons dans les selles humaines. tampon acide = Glycine 1M pH 2,5 et tampon R1 décrit dans l'exemple 2.**

| **Code Echantillon** | **Présence d'IgA** | **IgA anti-toxine B signal VIDAS® (RFV)** | |
|---|---|---|---|
| | | **Tampon R1 (état de l'art)** | **Tampon acide** |
| **S700** | Neg | 36 | 127 |
| **S706** | Neg | 9 | 60 |
| **S713** | Neg | 91 | 137 |
| **S720** | Neg | 56 | 169 |
| **S709** | Pos | 114 | 1303 |
| **S719** | Pos | 831 | 2797 |

| | | | |
|---|---|---|---|
| Pos = positif ; Neg = négatif | | | |

Le tampon acide glycine 1M pH 2,5 permet d'augmenter significativement les signaux RFV obtenus pour les échantillons positifs, de 3 à 7 fois plus qu'avec le tampon R1. La solution d'extraction à pH acide est choisie pour la suite des expériences.

### Exemple 4 : Etude du temps de contact entre l'échantillon et le tampon de traitement d'échantillon

La durée du contact entre l'échantillon et le tampon de traitement d'échantillon acide (condition de l'exemple 3) a été étudiée en utilisant un échantillon positif élevé (S719), un échantillon positif faible (S709) et un échantillon négatif (S700). Les résultats sont présentés dans la Figure 1. L'échantillon négatif (S700) n'a pas présenté de diminution significative du signal. Les échantillons positifs ont montré une diminution du signal. Dès 30 minutes d'incubation, il reste 91% du signal pour S719 et il reste 72% du signal pour S709. Au bout de 60 minutes, il reste 82% de signal pour S719 et 63% de signal pour S709. Ces résultats montrent qu'une incubation de 60 min en présence du tampon glycine 1M pH 2,5 fait diminuer de manière significative la quantité d'IgA anti-toxine B présente dans l'échantillon. Dans le cas d'échantillons faiblement positifs, il conviendrait de ne pas dépasser 15 min afin de ne pas perdre plus de 10% du signal. Un tel échantillon pourrait être détecté « négatif » si on prolonge trop la durée du contact avec le tampon d'extraction. Pour la suite des expériences, nous avons utilisé un temps de contact d'environ 30 secondes à 2 minutes.

### Exemple 5 : Comparaison toxine B recombinante et toxine B native pour la détection des IgA anti-toxine B par immunoessai

Nous avons comparé les signaux de fluorescence obtenus lors de l'utilisation d'une toxine B recombinante et d'une toxine B native pour la préparation de la phase de capture (adsorption sur les cônes). Les deux types de toxine B ont été immobilisés sur la phase solide à la même concentration (2 µg/mL). Les résultats sont présentés dans le Tableau 3 ci-dessous. Les échantillons SP023 et SP pool ont été obtenus auprès du Professeur M. Delmée, Centre National de Référence *Clostridium difficile,* Université Catholique de Louvain, Bruxelles, Belgique.

On a répété le mode opératoire décrit dans l'exemple 3 ci-dessus.

A l'exception de l'échantillon S709, les signaux VIDAS® obtenus pour tous les échantillons sont plus forts lorsque la phase solide présente la toxine native que la toxine recombinante. Pour les échantillons négatifs, le gain est négligeable (+37 à +67 RFV), alors que pour 3 des 4 échantillons positifs le gain est très important (+640 à +1797 RFV). Ainsi, l'utilisation de la toxine B native en capture permet une meilleure discrimination des échantillons positifs et négatifs.

D'après les résultats présentés dans le tableau 3, le seuil de positivité lorsqu'on utilise la toxine B recombinante est fixée à 200 RFV tandis que le seuil de positivité lorsqu'on utilise la toxine B native est fixée à 250 RFV.

**Tableau 3. Détection des IgA fécales anti-toxine B par la toxine B recombinante ou la toxine B native. Le pré-traitement des échantillons de selles a été effectué en présence du tampon acide glycine 1M pH 2,5, selon l'exemple 3.**

| **Code Echantillon** | **Présence d'IgA** | **IgA anti-toxine B signal VIDAS® (RFV)** | | **ΔSignal Native-Recomb**. |
|---|---|---|---|---|
| | | **Toxine B Recombinante** | **Toxine B Native** | |
| **S700** | Neg | 127 | 194 | +67 |
| **S706** | Neg | 60 | 97 | +37 |
| **S713** | Neg | 137 | 193 | +56 |
| **S720** | Neg | 169 | 215 | +46 |
| **S709** | Pos | 1303 | 623 | -680 |
| **S719** | Pos | 2797 | 3437 | +640 |
| **SP023** | Pos | 498 | 1221 | +723 |
| **SP pool** | Pos | 1637 | 3434 | +1797 |

| | | | | |
|---|---|---|---|---|
| Pos = positif ; Neg = négatif | | | | |

### Exemple 6 : Recherche d'IgA fécales chez les patients multirécidivistes et détermination des valeurs de référence S

Les échantillons de selles utilisés dans cet exemple ont été obtenus chez des patients ayant fait des infections à *Clostridium difficile* à répétition, appelés patients multirécidivistes. Ces patients ont consulté dans la Division de Microbiologie Clinique, dans le Département de Médecine de l'Hôpital Général Juif - Sir Mortimer B. Davis, à Montréal, Québec, Canada. Le pré-traitement des échantillons de selles a été effectué en présence du tampon acide glycine 1M pH 2,5, selon les conditions de l'exemple 3. Les différents types de toxine ont tous été immobilisés sur la phase solide à la même concentration (2 µg/mL). Les IgA fécales anti-toxine B, ainsi que les IgA fécales anti-toxine A ont été recherchées par immunoessai en utilisant les toxines décrites dans l'exemple 1 selon le mode opératoire décrit dans l'exemple 2. Les résultats sont présentés dans le Tableau 4.

**Tableau 4. Recherche des IgA fécales anti-toxine B et anti-toxine A dans les selles des patients multirécidivistes.**

| **Code Echantillon** | **Signal VIDAS® (RFV) IgA anti-toxine B** | | **Signal VIDAS® IgA anti-toxine A** |
|---|---|---|---|
| | **Toxine B Native** | **Toxine B Recombinante** | **Toxine A Recombinante** |
| **MR001** | 11 | 5 | 6 |
| **MR002** | 867 | 191 | 156 |
| **MR003** | 97 | 35 | 66 |
| **MR004** | 129 | 37 | 60 |
| **MR005** | 161 | 42 | 60 |
| **MR006** | 20 | 14 | NDi |
| **MR007** | 380 | 145 | 138 |
| **MR008** | 337 | 214 | NDi |
| **MR009*** | 1001 | 609 | 462 |

| | | | |
|---|---|---|---|
| NDi=non disponible, l'essai n'a pas pu être réalisé à cause d'un volume insuffisant d'échantillon. | | | |

Chez 3 patients multi-récidivistes parmi les 8 testés, il a été possible de mettre en évidence des IgA fécales anti-toxine B (MR002, MR007, MR008). Pour tous ces patients, le signal obtenu est supérieur au seuil de positivité du test de 250 RFV. De plus, ces résultats confirment de manière indépendante que l'immunoessai utilisant la toxine B recombinante est moins sensible que la méthode utilisant la toxine B native : le patient MR007 n'est pas détecté comme positif et le patient MR002 est en limite de positivité avec un signal de 191 RFV pour un seuil à 200 RFV.

Le suivi clinique des patients a montré que tous les patients MR001 à MR008 ont présenté au moins une récurrence après leur consultation et l'obtention des échantillons utilisés dans cet exemple. Nos tests montrent que certains de ces patients (MR002, MR007, MR008) ont des anticorps IgA anti-toxine B, et le cas échéant des anticorps IgA anti-toxine A, ou aucun de ces deux types d'anticorps. On peut conclure que le titre des anticorps IgA fécaux anti-toxine B, que ces patients possèdent, n'est pas suffisant pour protéger d'une telle récurrence. Ce groupe de patients constitue donc le groupe de patients à risque accru de développer ou redévelopper une infection à *Clostridium difficile.*

Le patient MR009 a été initialement classé dans le groupe des patients multirécidivistes d'après le tableau clinique. Les investigations biologiques complémentaires ont indiqué qu'il s'agit en fait d'une mauvaise classification. Ce patient a été suivi pendant 3 mois après ce premier test. La recherche de la bactérie *Clostridium difficile* par culture s'est toujours avérée négative malgré plusieurs tentatives. Aucune récurrence n'a été observée. Il convient donc de considérer que le patient MR009 est protégé de la récurrence. Ce patient a à la fois des IgA anti-toxine B (1001 RFV) et des IgA anti-toxine A (462 RFV). Ce patient constitue donc le groupe de patients n'étant pas à risque accru de développer ou redévelopper une infection à *Clostridium difficile.*

L'ensemble de ces résultats obtenus dans ces groupes de patients nous permettent alors de définir la valeur de référence S. Pour un antigène de capture donné, la valeur de référence S est choisie de telle manière que tous les signaux VIDAS® obtenus chez les patients multirécidivistes MR001 à MR008 lui sont inférieurs et ceux du patient MR009 lui sont supérieurs.

Pour les anticorps anti-toxine B, la valeur de référence S est entre 870 et 1000 RFV, soit fixée à environ 930 RFV, lorsqu'une toxine B native est utilisée en capture. Elle est entre 200 et 600 RFV, soit fixée à environ 400 RFV, lorsque la toxine B recombinante est utilisée en capture. Pour les anticorps anti-toxine A, la valeur de référence S est entre 160 et 460 RFV, soit fixée à environ 310 RFV, lorsqu'une toxine A recombinante est utilisée en capture.

### Exemple 7 : Recherche d'IgA fécales chez les patients toxines A et B négatifs en suspicion d'infection à Clostridium difficile

Les échantillons de selles utilisés dans cet exemple ont été obtenus chez des patients ayant consulté avec une suspicion d'infection à *Clostridium difficile,* dans le service du Dr. Robert Spencer, Heath Protection Agency Regional Laboratory, Bristol, Royaume-Uni. Les échantillons en notre possession ont été caractérisés en utilisant les trousses VIDAS® C. *difficile* GDH (Cat. No. 30125, bioMérieux) et VIDAS® C. *difficile* Toxines A et B (Cat. No. 30118, bioMérieux). La recherche des IgA fécales a été effectuée selon les protocoles décrits dans les exemples 2, 3 et 6 en utilisant la toxine B native et la toxine A recombinante.

Les échantillons sélectionnés pour l'étude de l'exemple 7 sont tous toxine A et toxine B négatifs, ainsi que GDH négatifs (Tableau 5). Cette sélection rassemble deux sous-groupes de patients. Dans un premier sous-groupe, ces deux tests sont négatifs parce que les patients n'ont jamais été en contact avec la bactérie *C*. *difficile.* Les symptômes qui ont évoqué une suspicion d'infection à *C*. *difficile* sont dus à une autre cause. Le deuxième sous-groupe correspond aux patients qui ont effectivement été en contact avec la bactérie mais qui ont pu l'éliminer ou limiter sa multiplication et ainsi guérir de leur infection. Nous n'avons pas les données cliniques qui nous permettraient de faire la distinction entre ces deux sous-groupes. Par contre, si nous mettons en évidence des IgA anti-toxine B et/ou des IgA anti-toxine A dans les selles des patients, il s'agit sans aucun doute de patients ayant fait une infection symptomatique ou porteur de *Clostridium difficile.* Ainsi, nous avons montré que 6 patients sur les 12 testés (50%) ont des taux d'anticorps anti-toxine B supérieurs à la valeur de référence S définie dans l'exemple 6. Parmi ces 6 patients, seulement 5 ont également des anticorps anti-toxine A qui sont présents et dont le taux est supérieur à la valeur de référence S définie dans l'exemple 6 (Tableau 5). Ainsi en recherchant uniquement des IgA anti-toxine A, le patient SP252 ne serait pas détecté comme ayant des anticorps sécrétoires anti-*Clostridium difficile.*

Les résultats obtenus sur cette cohorte montrent que la détection des IgA anti-toxine B est plus sensible que la détection des IgA anti-toxine A.

**Tableau 5. Recherche des IgA fécales anti-toxine B et anti-toxine A dans les selles de patients ayant une suspicion d'infection à Clostridium difficile.**

| **Code Echantillon** | **VIDAS® GDH** | **VIDAS® CDAB** | **Signal VIDAS® (RFV)** | | **Interprétation (par rapport à la valeur de référence)** | |
|---|---|---|---|---|---|---|
| | | | **IgA anti-toxine B** | **IgA anti-toxine A** | **IgA anti-toxine B** | **IgA anti-toxine A** |
| **SP29** | Neg | Neg | 2034 | 390 | Pos | Pos |
| **SP32** | Neg | Neg | 2094 | 1056 | Pos | Pos |
| **SP37** | Neg | Neg | 84 | 24 | Neg | Neg |
| **SP38** | Neg | Neg | 108 | 60 | Neg | Neg |
| **SP40** | Neg | Neg | 9684 | 4338 | Pos | Pos |
| **SP43** | Neg | Neg | 1044 | 378 | Pos | Pos |
| **SP228** | Neg | Neg | 24 | 18 | Neg | Neg |
| **SP232** | Neg | Neg | 12 | 18 | Neg | Neg |
| **SP234** | Neg | Neg | 42 | 24 | Neg | Neg |
| **SP236** | Neg | Neg | 2274 | 636 | Pos | Pos |
| **SP251** | Neg | Neg | 36 | 24 | Neg | Neg |
| **SP252** | Neg | Neg | 1026 | 240 | Pos | Neg |

| | | | | | | |
|---|---|---|---|---|---|---|
| Pos = positif ; Neg = négatif | | | | | | |

### Exemple 8 : Recherche d'IgA fécales chez les patients ayant une infection à Clostridium difficile et prédiction de la récurrence

Les échantillons de selles utilisés dans cet exemple ont été obtenus chez des patients ayant une infection à *Clostridium difficile* prouvée. Ces patients ont consulté dans la Division de Microbiologie Clinique, dans le Département de Médecine de l'Hôpital Général Juif - Sir Mortimer B. Davis, à Montréal, Québec, Canada. Quand cela a été possible, deux échantillons de selles consécutifs, séparés de 10 à 35 jours, ont été recueillis pour chaque patient. Le nombre de jours qui séparent le premier prélèvement du second sont indiqués dans le Tableau 6. Pour tous les patients, une recherche par PCR du matériel génétique de la bactérie *Clostridium difficile* a été effectuée sur les extraits des premiers prélèvements de selles. Cette recherche s'est avérée positive pour tous les patients inclus dans l'étude présentée ici, prouvant ainsi la suspicion clinique d'infection à *Clostridium difficile.*

La recherche des IgA fécales a été effectuée selon les protocoles décrits dans les exemples 2, 3 et 6 en utilisant la toxine B native. Les échantillons pour lesquelles on obtient un signal supérieur à la valeur de référence S déterminée dans l'exemple 6 (930 RFV quand la toxine B native est utilisée en capture) sont considérés comme positifs et ceux pour lesquels le signal est inférieur à cette valeur sont négatifs. Les résultats sont présentés dans le Tableau 6.

**Tableau 6. Recherche des IgA fécales anti-toxine B dans les selles des patients ayant une infection à Clostridium difficile.**

| | **1er prélèvement** | | **2ème prélèvement** | | | | | **Clinique** |
|---|---|---|---|---|---|---|---|---|
| **Code patient** | **Ig A anti-toxine B** | | **Jour** | **VIDAS® CDAB** | **Ig A anti-toxine B** | | **Risque de récurrence (prédiction)** | **Nombre de récurrences observées** |
| | **Signal RFV** | **Interprétation** | | | **Signal RFV** | **Interprétation** | | |
| CD001 | 10451 | Pos | NA | NDi | NDi | NDi | NDi | Pas de suivi |
| CD002 | 3623 | Pos | NA | NDi | NDi | NDi | NDi | 1 |
| CD003 | 413 | Neg | +13 | NDi | 2708 | Pos | Faible | 1 |
| CD004 | 675 | Neg | +12 | Pos | 3291 | Pos | Faible | 1 |
| CD005 | 1891 | Pos | NA | NDi | NDi | NDi | NDi | Pas de suivi |
| CD006 | 6881 | Pos | +19 | Pos | 2254 | Pos | Faible | 1 |
| CD007 | NDi | NDi | +34 | Pos | 7528 | Pos | Faible | 1 |
| CD008 | 3480 | Pos | +25 | NDi | 922 | Neg | Fort | 2 |
| CD009 | 2107 | Pos | +17 | Pos | 1475 | Pos | Faible | 1 |

| | | | | | | | | |
|---|---|---|---|---|---|---|---|---|
| Pos = positif; Neg = négatif; NDi=non disponible, l'essai n'a pas pu être réalisé à cause d'un volume insuffisant d'échantillon ; NA = non applicable | | | | | | | | |

Afin d'évaluer le risque de récurrence, il est utile de rechercher les IgA fécales anti-toxine B dans un prélèvement de selles obtenu 10 à 38 jours, de préférence 15 à 25 jours après l'infection par le *Clostridium difficile* considérée comme primaire.

Si le prélèvement de selles contient un taux d'IgA anti-toxine B supérieur à la valeur de référence S, alors le patient est à FAIBLE risque de récurrence : il fera 0 ou 1 récurrence maximum après l'épisode d'infection par le *Clostridium difficile* considérée comme primaire.

Si le prélèvement de selles contient un taux d'IgA anti-toxine B inférieur à la valeur de référence S, alors le patient est à FORT risque de récurrence : il fera 2 récurrences ou plus après l'épisode d'infection par le *Clostridium difficile* considérée comme primaire.

Les prédictions réalisées en appliquant cette règle sont présentées dans la colonne « Risque de récurrence » dans le Tableau 6 qui présente également le nombre de récurrence réellement observé chez ces mêmes patients. Nous pouvons remarquer qu'il y a une parfaite concordance entre les prédictions et les observations cliniques.

Pour 3 patients (CD001, CD002 et CD005), il n'a pas été possible d'obtenir un deuxième prélèvement 10 à 35 jours après le premier. Toutefois les analyses montrent que les premiers prélèvements de selles obtenus chez ces patients contiennent des taux importants d'IgA fécales anti-toxine B.

### Références Bibliographiques

- Bartlett, J.G., 2002, N. Engl. J. Med. 346 (5) : 334-339
- Bauer M.P. et al, 2011, Lancet, 14 : 63-73
- Eyre D.W. et al., 2012, Clinical Infectious Diseases, 55(S2) : 77-87
- Freeman J., 2010, Clin. Microbiol, 3 : 529-549
- Garey K.W. et al, 2008, Journal of Hospital Infection, 70 : 142-147
- Johnson S et al., 1992, JID, 166 : 1287-1294
- Kelly C.P. et Lamont. J.T., 2008, N Engl. J. Med., 359 : 1932-1940
- Kuijper E.J. et al, 2006, Clinical Microbiology and Infectious Diseases, CMI, 12(S6) : 2-18
- Tress U. et al., 2006, AJVR, 67(10) : 1756-1759
- Warny M. et al., 1994, Infection and Immunity, 62(2) : 384-389

## Revendications

1. Procédé de prédiction de la susceptibilité, pour un patient à risque, de développer ou redévelopper une infection à *Clostridium difficile*, comprenant la détermination par immunoessai, dans un échantillon de selles issu dudit patient, du taux d'anticorps IgA anti-toxine B de *Clostridium difficile*, et à comparer ce taux à une valeur de référence S préalablement déterminée avec deux populations de patients exposés à la bactérie, l'une n'ayant pas développé ou redéveloppé une telle infection et l'autre ayant développé ou redéveloppé une telle infection,
- un taux inférieur à ladite valeur de référence S signifiant que le patient est un patient à risque accru de développer ou redévelopper une infection à *Clostridium difficile,* et
- un taux supérieur à ladite valeur de référence S signifiant que le patient n'est pas un patient à risque accru de développer ou redévelopper une infection à *Clostridium difficile;*
et dans lequel, ledit échantillon de selles mis en œuvre dans l'immunoessai a été préalablement traité par un tampon de traitement d'échantillon acide, sans neutralisation avant sa mise en oeuvre dans l'immunoessai.

2. Procédé selon la revendication 1, dans lequel ledit tampon de traitement d'échantillon acide est à pH 2,5.

3. Procédé selon la revendication 1 ou 2, **caractérisé en ce que**, lors de l'étape de prétraitement de l'échantillon, l'échantillon est mis en contact avec le tampon de prétraitement d'échantillon acide pendant au plus 30 min, puis est soumis à une séparation, notamment par filtration ou sédimentation, le filtrat ou le surnageant étant alors récupéré pour sa mise en œuvre dans l'immunoessai.

4. Procédé de détermination, par immunoessai, du taux d'au moins un anticorps IgA dirigé contre la toxine B de *Clostridium difficile*, dans un échantillon biologique d'un patient susceptible de contenir ledit au moins un anticorps IgA, comprenant la mise en présence d'un ou plusieurs partenaires de liaison audit au moins un anticorps IgA, utilisé(s) pour la mise en œuvre de l'immunoessai, avec un milieu réactionnel acide comprenant ledit échantillon biologique prétraité par un tampon de traitement d'échantillon acide, sans neutralisation avant sa mise en œuvre dans l'immunoessai.

5. Procédé selon la revendication 4, **caractérisé en ce que** l'échantillon prétraité par le tampon acide est soumis à une séparation, notamment par filtration ou sédimentation, le filtrat ou le surnageant étant alors le milieu réactionnel récupéré pour sa mise en œuvre dans l'immunoessai,

6. Procédé selon la revendication 4 ou 5, **caractérisé en ce que** le tampon de prétraitement d'échantillon acide est à pH 2,5.

## Patentansprüche

1. Verfahren zur Vorhersage der Anfälligkeit eines Risiko-Patienten dafür, eine Infektion mit *Clostridium difficile* zu entwickeln oder erneut zu entwickeln, wobei es die Bestimmung des Gehalts an Antikörper IgA, der gegen das Toxin B von *Clostridium difficile* gerichtet ist, in einer Stuhlprobe, die von dem Patienten stammt, mittels Immunassay umfasst, woraufhin dieser Gehalt mit einem Bezugswert S verglichen wird, welcher im Vorfeld anhand zweier Populationen von Patienten bestimmt wurde, die dem Bakterium ausgesetzt waren, wobei eine davon keinerlei derartige Infektion entwickelt oder erneut entwickelt hat und die andere eine derartige Infektion entwickelt oder erneut entwickelt hat, wobei
- ein Gehalt unterhalb des Bezugswerts S bedeutet, es sich bei dem Patienten um einen Patienten handelt, der ein erhöhtes Risiko dafür aufweist, eine Infektion mit *Clostridium difficile* zu entwickeln oder erneut zu entwickeln, und
- ein Gehalt oberhalb des Bezugswerts S bedeutet, es sich bei dem Patienten nicht um einen Patienten handelt, der ein erhöhtes Risiko dafür aufweist, eine Infektion mit *Clostridium difficile* zu entwickeln oder erneut zu entwickeln;
und in welchem die Stuhlprobe, welche für den Immunassay eingesetzt wird, zuvor mit einem sauren Probenbehandlungspuffer behandelt worden ist, wobei vor ihrem Einsatz im Immunassay keine Neutralisierung erfolgt.

2. Verfahren nach Anspruch 1, wobei der saure Probenbehandlungspuffer einen pH-Wert von 2,5 hat.

3. Verfahren nach Anspruch 1 oder 2, **dadurch gekennzeichnet, dass** die Probe im Schritt des Vorbehandeln der Probe höchstens 30 min. lang mit dem sauren Probenvorbehandlungspuffer in Kontakt gebracht wird, woraufhin sie einem Trennvorgang unterzogen wird, insbesondere durch Filtration oder Absetzenlassen, wobei das Filtrat oder der Überstand gewonnen wird, um es/ihn im Immunassay einzusetzen.

4. Verfahren zur Bestimmung des Gehalts an mindestens einem Antikörper IgA, der gegen das Toxin B von *Clostridium difficile* gerichtet ist, mittels Immunassay, in einer biologischen Probe eines Patienten, die möglicherweise den mindestens einen Antikörper IgA enthält, wobei es das Zusammenbringen eines oder mehrerer Bindungspartner des mindestens einen Antikörpers IgA, wie sie für die Durchführung des Immunassays verwendet wird/werden, mit einem sauren Reaktionsmilieu umfasst, welches die biologische Probe umfasst, welche mit einem sauren Probenbehandlungspuffer vorbehandelt wurde, ohne dass vor ihrem Einsatz im Immunassay eine Neutralisierung erfolgt.

5. Verfahren nach Anspruch 4, **dadurch gekennzeichnet, dass** die Probe, welche mit dem sauren Puffer vorbehandelt wurde, einem Trennvorgang unterzogen wird, insbesondere durch Filtration oder Absetzenlassen, woraufhin das Filtrat oder der Überstand als Reaktionsmilieu gewonnen wird, um dieses dann im immunassay einzusetzen.

6. Verfahren nach Anspruch 4 oder 5, **dadurch gekennzeichnet, dass** der saure Probenvorbehandlungspuffer einen pH-Wert von 2,5 hat.

## Claims

1. A method for predicting the susceptibility of an at-risk patient for developing or redeveloping *Clostridium difficile* infection, comprising determining, by immunoassay, in a stool sample from said patient, the level of IgA antibodies against toxin B of *Clostridium difficile,* and comparing this level against a reference value S determined beforehand with two populations of patients exposed to the bacterium, one not having developed or redeveloped such an infection and the other having developed or redeveloped such an infection,
- a level below said reference value S signifying that the patient is a patient with increased risk of developing or redeveloping *Clostridium difficile* infection, and
- a level above said reference value S signifying that the patient is not a patient with increased risk of developing or redeveloping *Clostridium difficile* infection;
and wherein the stool sample used in the immunoassay had been treated beforehand with an acidic sample treatment buffer, without neutralization before it is used in the immunoassay.

2. The method as claimed in claim 1, wherein said sample treatment buffer is at pH 2.5.

3. The method as claimed in claim 2, **characterized in that**, in the sample pretreatment step, the sample is brought into contact with the acidic sample pretreatment buffer for at most 30 min, and it then undergoes separation, notably by filtration or sedimentation, the filtrate or supernatant then being recovered, to be used in the immunoassay.

4. A method for determining, by immunoassay, the level of at least one IgA antibody directed against against toxin B of *Clostridium difficile*, in a patient's biological sample that may contain said at least one IgA antibody, comprising bringing one or more binding partners to said at least one IgA antibody, used for performing the immunoassay, into contact with an acidic reaction mixture comprising said biological sample pretreated with an acidic sample treatment buffer, without neutralization before it is used in the immunoassay.

5. The method as claimed in claim 4, **characterized in that** the sample pretreated with the acidic buffer undergoes separation, notably by filtration or sedimentation, the filtrate or supernatant then being the reaction mixture recovered for use in the immunoassay.

6. The method as claimed in claim 4 or 5, **characterized in that** the acidic sample pretreatment buffer has pH 2.5.
